# EUROPÄISCHE PATENTSCHRIFT

(11) **EP 0 862 418 B1**
(45) Veröffentlichungstag und Bekanntmachung des Hinweises auf die Patenterteilung: **22.08.2001**
(21) Anmeldenummer: 96937286.1
(22) Anmeldetag: 30.10.1996
(51) Int. Cl.: A61K 9/16, A61K 9/50

(54) **VERFAHREN ZUR HERSTELLUNG VON MORPHOLOGISCH EINHEITLICHEN MIKROKAPSELN SOWIE NACH DIESEM VERFAHREN HERGESTELLTE MIKROKAPSELN**
METHOD OF PRODUCING MORPHOLOGICALLY UNIFORM MICROCAPSULES AND MICROCAPSULES PRODUCED BY THIS METHOD
PROCEDE POUR LA PRODUCTION DE MICROCAPSULES A MORPHOLOGIE UNIFORME ET MICROCAPSULES AINSI OBTENUES

(30) Priorität: 24.11.1995 DE 19545257
(43) Veröffentlichungstag der Anmeldung: 09.09.1998
(73) Patentinhaber: ACTIPAC Biosystems GmbH, 82152 Martinsried (DE)
(72) Erfinder: RÖSSLING, Georg, D-13465 Berlin (DE); ALBAYRAK, Celál, D-10999 Berlin (DE); TACK, Johannes, D-13595 Berlin (DE); SCHMITZ, Reinhard, D-10719 Berlin (DE)
(74) Vertreter: Tauchner, Paul, Dr.
(86) Internationale Anmeldenummer: EP9604701
(87) Internationale Veröffentlichungsnummer: WO9719676

(56) Entgegenhaltungen:
- EP-A- 0 190 833
- EP-A- 0 442 671

## Beschreibung

Die Erfindung betrifft ein Verfahren zur Herstellung von morphologisch einheitlichen Mikrokapseln, die Peptide, Proteine oder andere wasserlösliche biologisch aktive Substanzen als Wirkstoffe enthalten sowie nach diesem Verfahren hergestellte Mikrokapseln.

Bekanntermaßen stellen Peptide und Proteine Wirkstoffe mit großer Pharmakodynamik dar, die jedoch bei oraler Applikation wegen ihrer Hydrolyseempfindlichkeit im sauren Milieu des Magens sowie enzymatischen Abbau zersetzt und damit teilweise inaktiviert werden, so daß sich ihre Wirksamkeit im Gastrointestinaltrakt erheblich verringert.

Eine schnelle Inaktivierung von Proteinen und Peptiden ist jedoch auch nach parenteraler und speziell nach intravenöser Applikation aufgrund der oft sehr kurzen Halbwertszeit zu beobachten. Dies bedeutet, daß trotz großer Pharmakodynamik und theoretisch geringer therapeutischer Dosierungen Mehrfachgaben hoher Dosierungen notwendig werden können, die eine große Belastung für den Patienten bedeuten.

Geeignete Formulierungen,die die genannten Nachteile vermeiden, sind Depotsysteme in Form von Polymermikrokapseln oder Polymernanokapseln, die auch für Peptide zahlreich bekannt und in der Literatur beschrieben sind.

Sie besitzen die Vorteile, daß
- Peptide und Proteine vor rascher Inaktivierung geschützt sind,
- geringere Dosierungen pharmakologisch wirksam sind,
- Mehrfachgaben reduziert werden können,
- eine kontrollierte Freisetzung von Peptiden und Proteinen prinzipiell möglich ist,
- die verkapselten Wirkstoffe zielgerichtet transportiert und
- unerwünschte Nebenwirkungen reduziert werden können.

Die bekannten Verfahren zur Mikro - oder Nanoverkapselung von wasserlöslichen Substanzen können wie folgt eingeteilt werden:
- Koazervation bzw. Emulsionsphasentrennung
- Verkapselung durch Sprühtrocknung
- Solvent - Evaporation in organischer oder wäßriger Phase.

Alle Verfahren beinhalten die Einbettung der Wirkstoffe in eine bioabbaubare Polymermatrix bzw. Copolymermatrix.

Aus der Literatur bekannte Polymere für diesen Zweck sind Polyamide, Polyanhydride, Polyester, Polyorthoester, Polyacetate, Polylactone, Polyorthocarbonate u. a. Vor allem haben bisher Polylactid-co-Glycolid-Polymere Anwendung gefunden.

So sind z.B. aus US 4.675.189 (Syntex Inc.), US 4.835.139 (Debiopharm S.A.) und EP 302 582 B1 (Southern Research Inst.) pharmazeutische Zusammensetzungen wasserlöslicher Peptide und Proteine in Kapselform bekannt, die auf der Basis der Koazervation bzw. Emulsionsphasentrennung hergestellt wurden.

Gemäß dieser Offenbarung werden Verfahren beschrieben, bei denen das verwendete Copolymer, vorzugsweise Poly- (Lactid-co-Glycolid)-Polymer, in einem halogenierten organischen Lösungsmittel, bevorzugt Dichlormethan, gelöst wird, und in diese Lösung eine wäßrige Peptidlösung dispergiert wird. Danach wird ein sogenanntes Koazervations-Agenz zugesetzt. Das Koazervations-Agenz ist in dem organischen Lösungsmittel löslich, das Polymer ist jedoch unlöslich im Koazervations-Agenz, wodurch es zur Präzipitation des Polymeren unter Einschluß der dispergierten Polypeptide kommt. Als Koazervations-Agenz wird zur Phasentrennung üblicherweise Silikonöl eingesetzt. Nach Zugabe des Silikonöls muß außerdem noch eine große Menge Heptan zugegeben werden, das die Härtung der Mikrokapseln bewirkt.

Die Verkapselungseffizienz dieser Methode liegt bei ca. 70% (US 4.835.136). Die hergestellten Mikrokapseln weisen einen Durchmesser von 1 bis 500 *µ*m auf, gemäß der Beispiele vorzugsweise 10 bis 50*µ*m.

Die Nachteile dieses Verfahrens bestehen neben der Verwendung toxikologisch problematischer Mittel wie Dichlormethan, Heptan und Silikonöl auch in der Notwendigkeit des Einsatzes großer Lösungsmittelmengen, die aus der Verkapselung mittels Koazervations- Agentien, wie Silikonöl, resultiert.

Ein in EP-A 315875 (Hoechst AG) beschriebenes Verfahren zur Herstellung bioabbaubarer Mikrokapseln von wasserlöslichen Peptiden und Proteinen basiert auf dem Sprühtrocknungsverfahren, bei dem eine wäßrige Peptid- oder Proteinlösung in einer organischen Polymerlösung emulgiert und diese Emulsion sprühgetrocknet wird.

Als biologisch abbaubares Polymer wird eine Mischung aus Polyhydroxybuttersäure und Poly (Lactid-co-Glycolid) Polymer im Mischungsverhältnis zwischen 99:1 bis 20:80 eingesetzt.
Das Peptid oder Protein liegt in mikronisierter Form oder in wäßriger Lösung vor. Als Lösungsmittel kommen Chloroform, Dichlormethan, DMF oder ein Lösungsmittelgemisch aus Wasser /Ethanol /Chloroform in Betracht. Gemäß den Beispielen wird Chloroform eingesetzt. Die Sprühtrocknung erfolgt bei Temperaturen zwischen vorzugsweise 45 bis 95°C.

Nachteilig an diesem Verfahren ist die potentielle Explosionsgefahr bei Verwendung nichthalogenierter Lösungsmittel und der gleichzeitigen Anwendung höherer Temperaturen beim Trocknungsverfahren. Andererseits führt der Einsatz nichtentflammbarer Lösungsmittel wie Dichlorethan zu toxikologisch bedenklichen Restlösemittelkontaminationen im Endprodukt. Daneben zeigen sprühgetrocknete Mikrokapseln prinzipiell eine starke Agglomerationsneigung, es entstehen Agglomerate von ca. 100 µm Größe.

Nach dem "Solvent - Evaporation - Verfahren" hergestellte Mikropartikel sind in zwei kanadischen Patentanmeldungen CA 2.100.925 (Rhone-Merieux) und CA 2.099.941 (Tanabe Seiyaku Co.) beschrieben.

Üblicherweise wird bei dieser Methode die wäßrige Peptid- oder Proteinlösung in einer organischen Polymerlösung dispergiert, oder es werden Wirkstoffkristalle in der Polymerlösung suspendiert. Nach Zugabe einer zweiten wäßrigen Phase mit einer grenzflächenaktiven Substanz wird das Polymerlösungsmittel verdampft.

Diese Methode ist sehr variabel und es werden normalerweise W/O oder komplexe W/O/W-Emulsionen hergestellt.

Gemäß CA 2.099.941 werden wasserlöslicher Wirkstoff und bioabbaubares Polymer zuerst in einem Lösungsmittel oder einem Lösungsmittelgemisch gelöst, in dem sie beide löslich sind. Anschließend wird dieses Lösungsmittel entfernt und die entstandene feste Dispersion in einem mit Wasser nicht mischbaren organischen Lösungsmittel gelöst. Die resultierende Lösung (Ölphase) wird in einer wäßrigen Phase emulgiert, so daß eine W/O - Emulsion entsteht.

Zuletzt wird das organische Lösungsmittel der Ölphase dieser Emulsion verdampft.

EP-A- 0 442 671 beschreibt die Herstellung einer W/O/W Emulsion, wobei zunächst eine W/O Emulsion rubereitet wird, die anschließend einer wässrigen emulsatorhaltigen Phase zugesezt wird. Durch Entfernen der organischen Phase werden schließlich Mikrokapseln erhalten.

Konkrete Beispiele der Patentschrift betreffen Poly-(Lactid-co-Glycolid)-Polymer (PLGA) als Matrix und Thyreotropin freisetzendes Hormon (TRH) oder seine Derivate als Wirkstoff, die zuerst in einem Gemisch aus Acetonitril/Ethanol und ggf. Wasser, oder nur Acetonitril, oder aus Acetonitril und wäßriger Gelatine, oder aus Dichlormethan und Ethanol gelöst werden.

Als organische Lösungsmittel zur Lösung der festen Dispersion finden Dichlormethan oder Chloroform Anwendung. Eine wäßrige Polyvinyalkohol-Lösung stellt die wäßrige Phase dar.
Die Größe der Mikrokapseln liegt bei einem Durchmesser von 1 bis 100 µm, gemäß der konkreten Beispiele bei ca. 50 µm bis <100 *µ* m.

Gemäß CA 2.100.925 werden Mikrokapseln von LHRH-Hormon und Analoga durch vorheriges Dispergieren des LHRH-Hormons in Pulverform in zwei organischen Lösungsmitteln hergestellt, wobei das eine Lösungsmittel (genannt Dispersionslöungsmittel) das Herstellen einer homogenen Suspension des pulverisierten Hormons durch einfaches Rühren ermöglicht. Das zweite Lösungsmittel ist leicht mit Wasser mischbar und macht damit die Mikrodispersion der organischen Phase in der wäßrigen Phase möglich.

Als zweites Lösungsmittel wird Dichlormethan oder alternativ Chloroform verwendet. Die Kapseln weisen einen Durchmesser zwischen 1 bis 250 *µ*m auf. Vorzugsweise sind die Kapseln größer als 50 - 60 *µ*m.

Die Morphologie der so hergestellten Mikrokapseln ist ebenfalls sehr unterschiedlich. Wie oben bereits ausgeführt sind die verwendeten halogenierten Lösungsmittel toxikologisch bedenklich. Daneben benötigt dieses Verfahren auch größere Mengen grenzflächenaktiver Substanzen.

Aufgabe der Erfindung war es, ein einfaches und schonendes Verfahren zur Herstellung morphologisch einheitlicher, nicht agglomerierender Mikrokapseln unter Verwendung von toxikologisch unbedenklichen Lösungsmitteln zu entwickeln, das eine Verkapselungseffizienz von mindestens 85%, vorzugsweise über 90 %, aufweisen und Mikrokapseln im Größenbereich von 200 nm bis 500*µ*m mit hohem Beladungsgrad liefern soll. Außerdem soll das Verfahren ein "scaling up" ermöglichen.

Die Aufgabe der Erfindung wird überraschend einfach mittels der "Induced Phase Transition"-Methode gelöst, welche ausgeführt wird indem ein für die Mikrokapselherstellung übliches Polymer wie ein Polyester aus Hydroxycarbonsäuren oder ein Blockpolymer aus Polyestern von Hydroxycarbonsäuren und Polyethylenglykol (PEG) in einem halogenfreien, nicht oder partiell mit Wasser mischbaren Lösungsmittel oder Lösungsmittelgemisch gelöst wird und in diese Lösung die gepufferte Wirkstofflösung, die einen pH-Wert zwischen 6,0 - 8,0 hat, dispergiert wird. Durch Homogenisieren entsteht eine stabile W/O-Emulsion, zu der unter Rühren eine wäßrige Lösung enthaltend eine oberflächenaktive Substanz oder ein Gemisch oberflächenaktiver Substanzen als äußere Phase zugesetzt wird, so daß eine dreiphasige W/O/W-Emulsion erhalten wird. Nachfolgend wird das Lösungsmittel oder Lösungsmittelgemisch mit üblichen Methoden entfernt, vorzugsweise im Vakuum und/oder Luft/Stickstoffstrom. Die Mikrokapseln werden aufkonzentriert und ggf. gefriergetrocknet.

Die Partikelgröße wird dabei über die Rührgeschwindigkeit gesteuert, wobei kleinere Partikel (≤ 8*µ*m) - wie sie benötigt werden falls das Produkt für eine intravenöse Applikation bestimmt ist - bei höheren Rührgeschwindigkeiten erhalten werden.

Gewünschtenfalls werden die Mikrokapseln nach Entfernung des Lösungsmittels zusätzlich einer "cross-flow" Filtration unterzogen, wodurch sie von restlichem Tensid und Restlösemittel-Anteilen befreit werden. Dadurch gelingt es den "initial burst", d.h. eine hohe Wirkstoffabgabe unmittelbar nach der Applikation (durch an der Partikeloberfläche anhaftenden Wirkstoff), zu verringern bzw. zu vermeiden.

Zur Lyophilisation werden gegebenenfalls Kryoprotektoren wie Zucker, Zuckeralkohole oder Polyvinylpyrrolidonderivate zugesetzt.

Bevorzugte Polyester von Hydroxycarbonsäuren, die in dem erfindungsgemäßen Verfahren eingesetzt werden können, sind:
Polyglycolide (PGA)und Copolymere von Glycoliden wie Glycolid/Lactid Copolymere (PGA/PLLA) oder Glycolid/Trimethylencarbonat Copolymere (PGA/TMC); L-Polylactide (PLA) und Stereocopolymere von Polylactiden wie Poly-L-Lactid (PLLA), Poly-DL-Lactid Copolymere und L-Lactid /DL-Lactid Copolymere; Copolymere von PLA wie Lactid/Tetramethylglycolid Copolymere, Lactid/ δ-Valerolacton Copolymer und Lactid/ε-Caprolacton Copolymer; Poly-β-hydroxybutyrat (PHBA), PHBA/ β-Hydroxyvalerat Copolymere (PHBA/HVA), Poly-β-hydroxypropionat (PHPA), Poly-p-dioxanon (PDS), Poly-δ-valerolacton, hydrophobisierte Polysaccharide, - Hyaluronsäure, - Dextrane oder hydrophobisiertes Amylopektin und Poly-ε-caprolacton.

Als Blockcopolymere von Polyestern von Hydroxycarbonsäuren und linear- oder Star-Polyethylenglykol (PEG) können in dem erfindungsgemäßen Verfahren die nachfolgend genannten Anwendung finden:

AB-Blockcopolymere aus PLA und PEG, ABA-TriblockCopolymere aus PLA-PEG-PLA, S(3)-PEG-PLA Blockcopolymere und S(4)-PEG-PLA Blockcopolymere.

Bevorzugt wird gemäß der Erfindung das Polymer Resomer® 505 insbesondere Resomer® RG-756 oder Resomer® RG-858 eingesetzt.

Resomer® ist eine Marke der Firma Böhringer Ingelheim Es handelt sich dabei um ein (DL-Lactid-co-glycolid)-Polymer.

Erfindungsgemäß bevorzugte halogenfreie Lösungsmittel oder Lösungsmittelgemische sind Aceton, Ethanol, Alkylacetate wie Methyl-, Ethyl-, Propyl-, Isopropyl- oder Butylacetat, Alkylformiate wie Methyl-, Ethyl-, Propyl-, Isopropyl- oder Butylformiat, Triacetin, Triethylcitrat und/oder C₁-C₄ - Alkyllactate z. B. Methyl- oder Ethyllactat.

Besonders bevorzugt werden Ethylacetat, Isopropylacetat und Propylformiat eingesetzt.

Gepufferte Lösungen im Sinne der Erfindung sind wäßrige Lösungen von Peptiden, Proteinen bzw. von deren physiologisch verträglichen Salzen oder von anderen wasserlöslichen biologisch aktiven Substanzen, die vorzugsweise mit einer Tris (hydroxymethyl) aminomethanlösung oder einer Phosphatpufferlösung auf einen pH-Wert zwischen 6,0 und 8,0, vorzugsweise pH 6,5 bis 7,4, eingestellt werden.

Ein weiterer erfindungsgemäß eingesetztbarer Puffer ist der Citratpuffer, wobei die Pufferkonzentrationen im allgemeinen im Bereich von 5mmol/l bis 300 mmol/l liegen.

Mit dem erfindungsgemäßen Verfahren können beliebige wasserlösliche Peptide bzw. Proteine verkapselt werden. Insbesondere geeignet ist das erfindungsgemäße Verfahren zur Verkapselung von Humanserumalbumin, Insulin, Interferone und LHRH-Antagonisten oder deren Analoga.

Ganz besonders vorteilhaft können mit dem erfindungsgemäßen Verfahren morphologisch einheitliche Mikrokapseln von Humanserumalbumin, Insulin, Interferonen und den nachfolgend genannten Peptiden hergestellt werden:
a) DesA(2)Nal-beta-Ala-DCpa-DPal-Ser-Tyr-DCit-Leu-Arg-Pro-DAla-NH₂,
b) DesA(2)Nal-Gly-DCpa-DPal-Ser-Tyr-DCit-Leu-Arg-Pro-DAla-NH₂,
c) Ac-DNal-DCpa-DPal-Ser-Tyr-DLys(Mor)-Leu-Lys(Mor)Pro-DAla-NH₂.

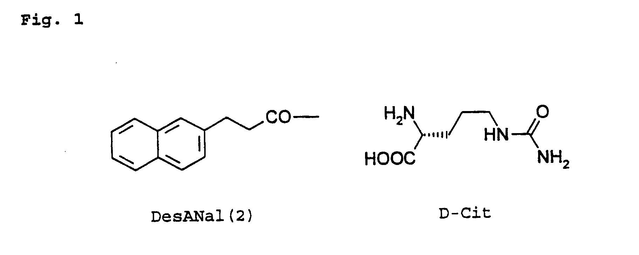

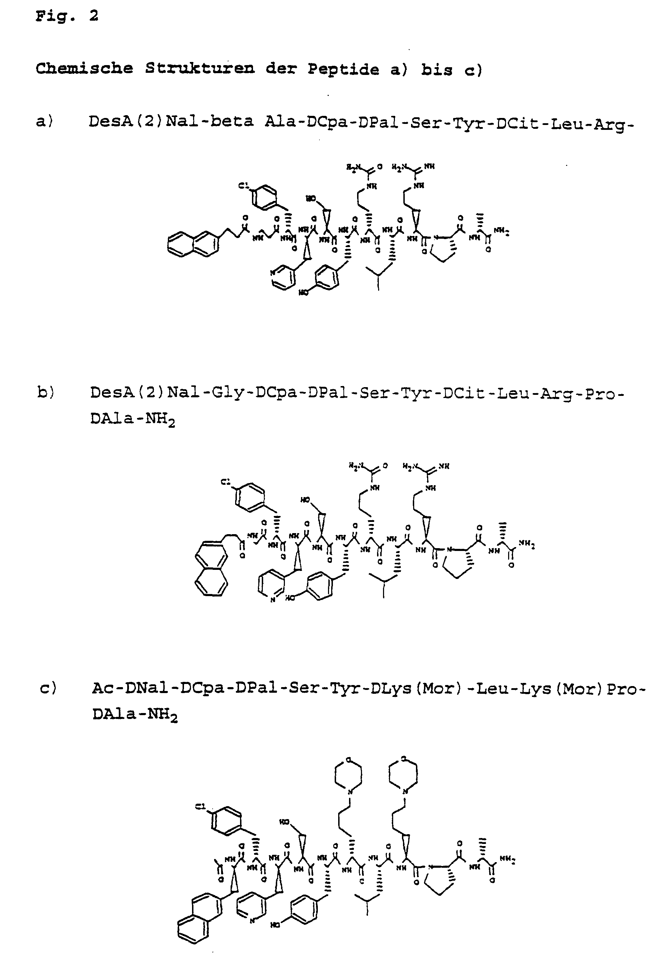

Die Bedeutungen von DesA(2)Nal und D-Cit und die chemischen Strukturen der Peptide a) bis c) sind in Figur 1 bzw. 2 aufgeführt.

Im Sinne der Erfindung werden als oberflächenaktive Substanzen bevorzugt Substanzen aus der Poloxamere® Gruppe, Polyethylenglycol Alkylether, Polysorbate (Tween®, Span®), Saccharoseester (Sisterna®, the Netherlands), Saccharoseester (Ryoto sugar ester, Tokyo), Gelatine, Polyvinylpyrrolidon, Fettalkoholpolyglycosid, Charps, Charpso, Decyl-β-D-Glycopyranosid, Decyl-β-D-Maltopyranosid, Dodecyl-β-D-Maltopyranosid, Natrium-oleat, Poloxamine® Gruppe, Polyethylenglykol, Polyvinyalkohol, polyoxyethylierte Fettsäureether (Brij®),Triton X 100 oder deren Gemische.

Bevorzugt finden Polyvinylalkohol, Brij®, Poloxamere® , Poloxamine® und Tween® Anwendung.

Gegenstand der Erfindung sind auch morphologisch einheitliche Mikrokapseln, die nach dem genannten Verfahren hergestellt werden und einen Durchmesser von 200nm bis 500*µ*m, vorzugsweise zwischen 0,2 bis 8 *µ*m aufweisen.

Aufgrund der günstigen Konstellation von Polymer und Lösungsmittel kommt es bei dem erfindungsgemäßen Verfahren zu keiner Bildung von Agglomeraten der Mikrokapseln.

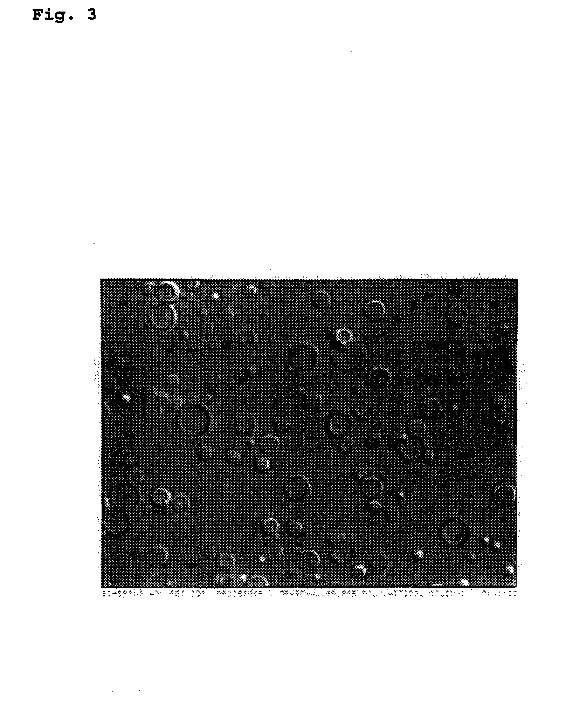

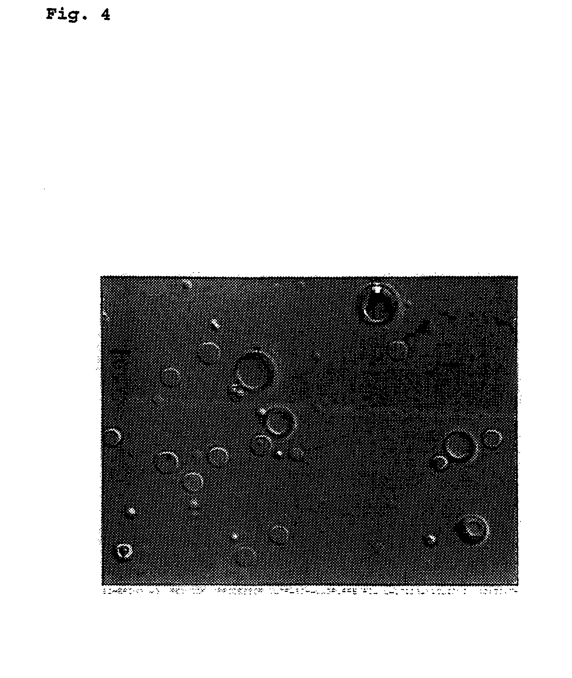

So zeigen Figuren 3 und 4 lichtmikroskopische Aufnahmen der erfindungsgemäßen Mikrokapseln hergestellt nach Beispiel 10 (Fig. 3) bzw. nach Beispiel 15 (Fig. 4). Ein Millimeter in der Abbildung entspricht 1 *µ*m in Realität. Die Aufnahmen zeigen deutlich die einheitliche Morphologie, Partikelagglomerate liegen nicht vor.

Die Verkapselungseffizienz des Verfahrens liegt bei mindestens 85%, vorzugsweise werden Verkapselungseffizienzen zwischen 90 - 95 % erreicht. Als Verkapselungseffizienz ist die Masse des verkapselten Wirkstoffes · 100/Masse des eingesetzten Wirkstoffes zu verstehen. Der Beladungsgrad der hergestellten Mikrokapseln liegt zwischen 3 bis 30 % (Beladungsgrad = Wirkstoffmasse · 100 / Wirkstoffmasse + Polymermasse).

Anschließend soll die Erfindung an Ausführungsbeispielen näher erläutert werden, ohne sie darauf einzuschränken.

### Beispiel 1

1,7 g des Polymers Resomer ® RG-756 werden in 29 ml Ethylaceat gelöst und in ein Stahlgefäß (Höhe 11,5 cm, Innendurchmesser 8 cm) überführt. Anschließend werden 3 ml einer wäßrigen, 200 mg Humanalbumin enthaltenden, 5 mmolaren Tris(hydroxymethyl)aminomethanlösung (pH 7,4) mit Hilfe eines mechanischen Rührers (Dispermat-FT, VMA-Getzmann GmbH, 5cm Dissolverscheibe) in der Polymerlösung 6 min bei 10000 U/min unter Raumtemperatur dispergiert. Zu der entstandenen W/O- Emulsion werden unter Rühren (8000 U/min) 45 ml einer wäßrigen Lösung, bestehend aus einer 2 % igen Polyvinylalkohollösung (Molekulargewicht 9000 - 10000, Aldrich) gegeben. Nach einer Dispergierzeit von 10 sec. wird die W/O/W-Emulsion in einen 500 ml Dreihalskolben überführt und mittels KPG-Rührer gerührt. Das Lösungsmittel Ethylacetat wird dann unter 20°C durch Anlegen eines Vakuums (900 mbar), Stickstoff- oder Lufteinleitung entfernt. Nach 5 h wird die Suspension mit 5 l Wasser oder einer wäßrigen Lösung gewaschen und auf ein gewünschtes Suspensionsvolumen eingeengt. Es wird eine "cross flow" Filtration mittels eines Sartocon Mini ® (Sartorius AG, Göttingen) Systems durchgeführt. Die lösungsmittel- und annähernd emulgatorfreie Suspension wird mit einem Kryoprotektor (beispielsweise mit einem Zucker, Zuckeralkohol oder Polyvinylpyrrolidonderivat) versetzt, möglichst schnell, beispielsweise mit flüssigem Stickstoff, eingefroren und gefriergetrocknet.

Das mit Wasser oder mit einer wäßrigen Lösung resuspendierte Lyophilisat enthält Mikrokapseln mit einem Humanalbumingehalt von 9 % (Humanalbuminmasse · 100 / Humanalbuminmasse + Polymermasse = Beladungsgrad) und sie besitzen einen Durchmesser von 0,2 bis 8 *µ*m. Die Verkapselungseffizienz beträgt 86 %.

### Beispiel 2

Es wird wie in Beispiel 1 verfahren, wobei 1,7 g Resomer ® RG-756 nicht in 29 ml. Ethylacetat, sondern in 40 ml Methylacetat gelöst werden.

### Beispiel 3

Es wird wie in Beispiel 1 verfahren, wobei statt 1,7 g des Polymers Resomer ® RG-756 1,1 g des Polymers Resomer ® RG-858 verwendet wird.

### Beispiel 4

Es wird wie in Beispiel 1 verfahren, wobei statt 1,7 g des Polymers Resomer ® RG-756 3,0 g des Polymers Resomer ® RG-858 verwendet werden.

### Beispiel 5

Es wird wie in Beispiel 1 verfahren, wobei statt einer 2 % PVA Lösung eine 2 % Brij ® 35 Lösung verwendet wird.

### Beispiel 6

Es wird wie in Beispiel 1 verfahren, wobei statt einer 2 % PVA Lösung eine 2 % Brij **®** 96 Lösung verwendet wird.

### Beispiel 7

Es wird wie in Beispiel 1 verfahren, wobei statt einer 2 % PVA Lösung eine 2 % Tween ® 20 Lösung verwendet wird.

### Beispiel 8

1,1 g des Polymers Resomer ® RG-858 werden in 29 ml Ethylacetat gelöst und in ein Stahlgefäß (Höhe 11,5 cm, Innendurchmesser 8 cm ) überführt.
Anschließend werden 7 ml einer 50 mg des Peptides DesA(2)Nal-beta-Ala-DCpa-DPal-Ser-Tyr-DCit-Leu-Arg-Pro-DAla-NH₂ (Peptid a) und 2 ml Ethanol enthaltenden, 5 mmolaren Tris (hydroxymethyl) aminomethanlösung (pH 7,4) mit Hilfe eines mechanischen Rührers (Dispermat-FT, VMA-Getzmann GmbH, 5 cm Dissolverscheibe) in der Polymerlösung 6 min bei 10000 U/min unter Raumtemperatur dispergiert. Zu der entstandenen W/O- Emulsion werden unter Rühren (8000 U/min) 45 ml einer wäßrigen Lösung, bestehend aus einer 2%igen Polyvinyalkohollösung (Molekulargewicht 9000-10000, Aldrich) gegeben. Nach einer Dispergierzeit von 10 sec. wird die W/O/W-Emulsion in einen 500 ml Dreihalskolben überführt und mittels KPG-Rührer gerührt. Das Lösungsmittel Ethylacetat wird dann unter 20°C durch Anlegen eines Vakuums (900 mbar), Stickstoff- oder Lufteinleitung entfernt. Nach 5 h wird die Suspension mit 5 l Wasser oder einer wäßrigen Lösung gewaschen und auf ein gewünschtes Suspensionsvolumen eingeengt. Es wird eine "cross flow" Filtration mittels eines Sartocon Mini ® (Sartorius AG, Göttingen) Systems mit einer Polyolefinmembran (cutoff 0,2 *µ*m) durchgeführt. Die lösungsmittel- und annähernd emulgatorfreie Suspension wird mit einem Kryoprotektor (beispielsweise mit einem Zucker, Zuckeralkohol oder Polyvinylpyrrolidonderivat) versetzt, möglichst schnell, beispielsweise mit flüssigem Stickstoff, eingefroren und gefriergetrocknet.

Das mit Wasser oder mit einer wäßrigen Lösung resuspendierte Lyophilisat enthält Mikrokapseln mit einem Wirkstoffgehalt von 4 %. Die Mikrokapseln besitzen einen Durchmesser von 0,2 bis 8 *µ* m. Die Verkapselungseffizienz beträgt 93 %.

### Beispiel 9

1,1 g des Polymers Resomer ® RG-858 werden in 29 ml Ethylacetat gelöst und in ein Stahlgefäß (Höhe 11,5 cm, Innendurchmesser 8 cm) überführt. Anschließend werden 5 ml einer wäßrigen, 48 mg des Peptids DesA(2)Nal-Gly-DCpa-DPal-Ser-Tyr-DCit-Leu-Arg-Pro-DAla-NH₂ (Peptid b) enthaltenden, 5 mmolaren Tris(hydroxymethyl)amino-methanlösung (pH 7,4) mit Hilfe eines mechanischen Rührers (Dispermat-FT, VMA-Getzmann GmbH, 5 cm Dissolverscheibe) in der Polymerlösung 6 min bei 10000 U/min unter Raumtemperatur dispergiert. Zu der entstandenen W/O-Emulsion werden unter Rühren (8000 U/min) 45 ml einer wäßrigen Lösung, bestehend aus einer 2 %igen Polyvinylalkohollösung (Molekulargewicht 9000-10000, Aldrich) gegeben. Nach einer Dispergierzeit von 10 sec. wird die W/O/W-Emulsion in einen 500 ml Dreihalskolben überführt und mittels KPG-Rührer gerührt. Das Lösungsmittel Ethylacetat wird dann unter 20 °C durch Anlegen eines Vakuums (900 mbar), Stickstoff- oder Lufteinleitung entfernt. Nach 5 h wird die Suspension mit 5 l Wasser oder einer wäßrigen Lösung gewaschen und auf ein gewünschtes Suspensionsvolumen eingeengt. Vorteilhaft ist der Einsatz einer "cross flow" Filtration, beispielsweise mit einem Sartocon Mini ® (Sartorius AG, Göttingen) System mit einer Polyolefinmembran (cutoff 0,2 *µ*m). Die lösungsmittel- und annähernd emulgatorfreie Suspension kann mit einem Kryoprotektor (beispielsweise mit einem Zucker, Zuckeralkohol oder Polyvinylpyrrolidonderivat) versetzt werden und wird möglichst schnell, beispielsweise mit flüssigem Stickstoff, eingefroren und gefriergetrocknet.

Das mit Wasser oder mit einer wäßrigen Lösung resuspendierte Lyophilisat enthält Mikrokapseln mit einem Wirkstoffgehalt von 4 %. Die Mikrokapseln besitzen einen Durchmesser von 0,2 bis 8 *µ*m. Die Verkapselungseffizienz beträgt 95,7 %.

### Beispiel 10

1,1 g des Polymers Resomer ® RG-858 werden in 30 ml Propylformiat gelöst und in ein Stahlgefäß (Höhe 11,5 cm, Innendurchmesser 8 cm) überführt. Anschließend werden 5 ml einer wäßrigen, 50 mg des LHRH Antagonisten Ac-DNal-DCpa-DPal-Ser-Tyr-DLys(Mor)-Leu-Lys(Mor)Pro- DAla-NH₂ (Peptid c) enthaltenden, 5 mmolaren
Tris(hydroxymethyl)aminomethanlösung (pH 7,0) mit Hilfe eines mechanischen Rührers (Dispermat-FT, VMA-Getzmann GmbH, 5 cm Dissolverscheibe) in der Polymerlösung 6 min bei 10000 U/min unter Raumtemperatur dispergiert. Zu der entstandenen W/O Emulsion werden unter Rühren (8000 U/min) 45 ml einer wäßrigen Lösung, bestehend aus einer 2 % igen Polyvinylalkohollösung (Molekulargewicht 9000-10000, Aldrich), gegeben. Nach einer Dispergierzeit von 10 sec. wird die W/O/W-Emulsion in einen 500 ml Dreihalskolben überführt und mittels KPG-Rührer gerührt. Das Lösungsmittel Propylformiat wird dann unter 20 °C durch Anlegen eines Vakuums (900 mbar), Stickstoff- oder Lufteinleitung entfernt. Nach 5 h wird die Suspension mit 5 l Wasser oder einer wäßrigen Lösung gewaschen und auf ein gewünschtes Suspensionsvolumen eingeengt. Es erfolgt eine "cross flow" Filtration mit einem Sartocon Mini ® (Sartorius AG, Göttingen) System mit einer Polyolenfinmembran (cutoff 0,2 *µ*m). Die Lösungsmittel- und annähernd emulgatorfreie Suspension wird möglichst schnell mit flüssigem Stickstoff eingefroren und gefriergetrocknet.

Das mit Wasser oder mit einer wäßrigen Lösung resuspendierte Lyophilisat enthält Mikrokapseln mit einem Wirkstoffgehalt von 3,9 %, und die Mikrokapseln besitzen einen Durchmesser von 0,2 bis 8 *µ*m. Die Verkapselungseffizienz beträgt 90,7 %.

### Beispiel 11

1,5 g des Polymers Resomer ® RG-858 werden in 30 ml Isopropylacetat gelöst und in ein Stahlgefäß (Höhe 11,5 cm, Innendurchmesser 8 cm) überführt. Anschließend werden 5 ml einer wäßrigen, 50 mg LHRH Antagonist wie Beispiel 10 enthaltenden, 5 mmolaren Tris (hydroxymethyl) aminomethanlösung (pH 7,0) mit Hilfe eines mechanischen Rührers (Dispermat-FT, VMA-Getzmann GmbH, 5 cm Dissolverscheibe) in der Polymerlösung 6 min bei 10000 U/min unter Raumtemperatur dispergiert.
Zu der entstandenen W/O-Emulsion werden unter Rühren (8000 U/min) 45 ml einer wäßrigen Lösung, bestehend aus einer 2 % igen Polyvinylalkohollösung (Molekulargewicht 9000-10000, Aldrich) gegeben. Nach einer Dispergierzeit von 10 sec. wird die W/O/W-Emulsion in einen 500 ml Dreihalskolben überführt und mittels KPG-Rührer gerührt. Das Lösungsmittel Isopropylacetat wird dann unter 20 °C durch Anlegen eines Vakuums (900 mbar), Stickstoff- oder Lufteinleitung entfernt. Nach 5 h wird die Suspension mit 5 l Wasser oder einer wäßrigen Lösung gewaschen und auf ein gewünschtes Suspensionsvolumen eingeengt. Es wird eine "cross flow" Filtration mit einem Sartocon Mini ® (Sartorius AG, Göttingen) System mit einer Polyolefinmembran (cutoff 0,2 *µ*m) durchgeführt und die Lösungsmittel- und annähernd emulgatorfreie Suspension wird lyophilisiert.

Das mit Wasser oder mit einer wäßrigen Lösung resuspendierte Lyophilisat enthält Mikrokapseln mit einem Wirkstoffgehalt von 2,9 % und die Mikrokapseln besitzen einen Durchmesser von 0,2 bis 8 *µ*m. Die Verkapselungseffizienz beträgt 90,6 %.

### Beispiel 12

Es wird wie in Beispiel 1 verfahren, wobei die 5 mmolare Tris(hydroxymethyl)aminomethanlösung (pH 7,0) durch eine 5 mmolare Phosphatpufferlösung (PBS, pH 7,2) ersetzt wird.

### Beispiel 13

Es wird wie in Beispiel 1 verfahren, wobei statt 200 mg HSA gelöst in 3 ml Trispuffer (pH = 7,4) 750mg HSA gelöst in 5 ml Trispuffer (pH = 7,4) eingesetzt wird.

Das in Wasser oder wäßrigen Lösungen resuspendierte Lyophilisat enthält Mikrokapseln mit einem HSA-Gehalt von 30%. Die Verkapselungseffizienz beträgt 90,9%.

### Beispiel 14

Es wird wie in Beispiel 13 verfahren, wobei anstelle der 2 %igen Polyvinylalkohollösung eine 2 %iger Poloxamer F 127 Lösung eingesetzt wird.

### Beispiel 15

Es wird wie in Beispiel 13 verfahren, wobei anstelle der 2 %igen Polyvinylalkohollösung eine 2 %iger Poloxamin T 707 Lösung eingesetzt wird.

### Beispiel 16

Es wird wie in Beispiel 13 verfahren, wobei anstelle der 2 %igen Polyvinylalkohollösung eine 2 %iger Poloxamin T 908 Lösung eingesetzt wird.

### Beispiel 17

Es wird wie in Beispiel 1 verfahren, wobei 200 mg HSA durch 200 mg Insulin (Human, Recombinant (pfs), Sigma Chemie Nr. I 0259) ersetzt werden.

### Beispiel 18

Es wird wie in Beispiel 1 verfahren, wobei 200 mg HSA durch 200 mg Interferon (Human Leukocyte (pfs)(α-IFH, Le), Sigma Chemie Nr. I 1008) ersetzt werden.

### Beispiel 19

Es wird wie in Beispiel 1 verfahren, wobei 200 mg HSA durch 200 mg Insulin (Human, gamma (pfs)(γ-IFN), Sigma Chemie Nr. I 6507) ersetzt werden.

### Beispiel 20

120 mg Insulin werden in 0.8 ml HCl (0,1 N) gelöst und mit 2 ml NaCl-Lösung (0,9 %) versetzt. Anschließend der pH-Wert der Lösung mit NaOH (0,1 N) auf 6-7 eingestellt. Diese Lösung wird zu einer Lösung von 500 mg Polymer RG-858 in 10 ml Ethylacetat gegeben und anschließend mit Ultraturax (bei 10.000-15.000 UPM) 3-4 Minuten gerührt. Danach werden unter Rühren 50 ml einer 2 %igen wäßrigen Polaxamin T707 Lösung zugegeben. Nach erfolgter Zugabe wird die Suspension in einen Dreihals-Kolben, der mit einem Rührer versehen ist, überführt. Durch Anlegen eines Vakuums wird unter Rühren das Lösungsmittel (Ethylacetat) entfernt. Der verbleibende Rückstand wird in einer Cross-flow Filtration (Sartocon Mini® Sartorius AG, Göttingen ) mit 5 Litern Wasser gewaschen. Der nahezu lösungsmittel- und tensidfreie Mikrokapseln enthaltende Rückstand wird, gegebenenfalls unter Zugabe eines Kyroprotektors, schnell eingefroren und gefriergetrocknet.

Das mit Wasser oder mit einer wäßrigen Lösung resuspendierte Lyophilisat enthält Mikrokapseln mit einem Beladungsgrad von 15 Gewichtsprozent.

### Beispiel 21

Es wird wie in Beispiel 20 verfahren, wobei anstelle der 2 %igen Polaxamin T707 Lösung, eine 2 %ige Polaxamer -407 (F127) Lösung eingesetzt wird.

Der Beladungsgrad der erhaltenen Mikrokapseln beträgt 17%.

### Beispiel 22

Es wird wie in Beispiel 20 verfahren, wobei anstelle der 2 %igen Polaxamin T707 Lösung, eine 2 % ige Polaxamer-188 (F68) Lösung eingesetzt wird.

Der Beladungsgrad der erhaltenen Mikrokapseln beträgt 16%.

### Beispiel 23

Es wird analog zu Beispiel 20 verfahren. Jedoch werden hier 700 mg Polymer und 223 mg Insulin eingesetzt. Das in Wasser oder einer wäßrigen Lösung resuspendierte Lyophilisat enthält Mikrokapseln mit einem Insulingehalt von 20 %.

## Patentansprüche

1. Verfahren zur Herstellung von morphologisch einheitlichen Mikrokapseln aus bioabbaubaren Polymeren oder Copolymeren, die Peptide, Proteine oder andere wasserlösliche biologisch aktive Substanzen als Wirkstoff enthalten, nach der "Induced Phase Transition"-Methode
dadurch gekennzeichnet, daß
- Polyester von Hydroxycarbonsäuren oder Blockcopolymere aus Polyestern von Hydroxycarbonsäuren und Polyethylenglykol in einem halogenfreien, nicht oder partiell mit Wasser mischbaren Lösungsmittel oder Lösungsmittelgemisch gelöst werden,
- in diese Lösung die gepufferte Wirkstofflösung, die einen pH-Wert zwischen 6,0 bis 8,0 besitzt, dispergiert wird,
- anschließend zu dieser W/O-Emulsion eine wäßrige Lösung enthaltend eine oberflächenaktive Substanz oder ein Gemisch oberflächenaktiver Substanzen zugesetzt wird,
- danach das Lösungsmittel oder ösungsmittelgemisch in üblicher Art und Weise entfernt wird und
- wahlweise verbliebenes lösungsmittel nicht mikroverkapseller Wirkstoff und/oder oberflächenaktive Substanz entfernt werden.

2. Verfahren nach Anspruch 1,
dadurch gekennzeichnet,
daß als halogenfreie Lösungsmittel Aceton, Ethanol; C₁-C₄-Alkylacetate, Triacetin, Triethylcitrat, C₁-C₄-Alkylformiate und C₁-C₄-Alkyllactate oder deren Gemische eingesetzt werden.

3. Verfahren nach einem der Ansprüche 1 oder 2,
dadurch gekennzeichnet,
daß als halogenfreie Lösungsmittel Methylacetat, Ethylacetat, Isopropylacetat und Propylformiat verwendet werden.

4. Verfahren nach einem der Ansprüche 1 bis 3,
dadurch gekennzeichnet,
daß als Pufferlösung eine Phosphatpufferlösung, eine Citratpufferlösung oder eine Tris(hydroxymethyl)aminomethanlösung eingesetzt wird.

5. Verfahren nach einem der Ansprüche 1 bis 4,
dadurch gekennzeichnet,
daß die gepufferte Wirkstofflösung eine pH-Wert zwischen 6,0 bis 8,0 aufweist.

6. Verfahren nach einem der Ansprüche 1 bis 4,
dadurch gekennzeichnet,
daß als Polymer Resomer® eingesetzt wird, vorzugsweise Resomer® RG-756 oder Resomer® RG-858.

7. Verfahren nach einem der Ansprüche 1 bis 6,
dadurch gekennzeichnet,
daß als Wirkstoff Humanserumalbumin, Peptide, Proteine, Interferone, Betaseron®, Insulin, LHRH-Antagonisten oder deren Analoga eingesetzt werden.

8. Verfahren nach einem der Ansprüche 1 bis 7,
dadurch gekennzeichnet,
daß als Wirkstoff
a) DesA(2)Nal-beta Ala-DCpa-DPal-Ser-Tyr-DCit-Leu-Arg-Pro-DAla-NH₂
b) DesA(2)Nal-Gly-DCpa-DPal-Ser-Tyr-DCit-Leu-Arg-Pro-DAla-NH₂
oder
c) Ac-DNal-DCpa-DPal-Ser-Tyr-DLys(Mor)-Leu-Lys(Mor)Pro-DAla-NH₂
Verwendung finden.

9. Verfahren nach einem der Ansprüche 1 bis 8, dadurch gekennzeichnet, daß die Entfernung des Lösungsmittels bzw. Lösungsmittelgemisches, sowie des gegebenenfalls an der Partikeloberfläche anhaftenden Wirkstoffs und/oder Tensids in einer "cross-flow" Filtration erfolgt.

10. Morphologisch einheitliche Mikrokapseln mit einem Beladungsgrad zwischen 3 bis 30 Gew.% und einem Durchmesser 200 nm bis 500*µ*m hergestellt nach dem Verfahren gemäß der Ansprüche 1 bis 9.

11. Mikrokapseln gemäß Anspruch 10 mit einem Durchmesser zwischen 0,2 bis 8 *µ*m.

## Claims

1. Process for the production of morphologically uniform microcapsules from biodegradable polymers or copolymers according to the "induced phase-transition" method, the said microcapsules containing peptides, proteins or other water-soluble, biologically active substances as the active ingredient,
**characterised in that**
- polyesters of hydroxycarboxylic acids or block copolymers of polyesters of hydroxycarboxylic acids and polyethylene glycol are dissolved in a halogen-free solvent or solvent mixture which is immiscible or partially miscible with water,
- the buffered active ingredient solution, which has a pH between 6.0 and 8.0, is dispersed in this solution,
- subsequently, an aqueous solution containing a surfactant or a mixture of surfactants is added to this water/oil emulsion,
- the solvent or mixture of solvents is then removed by usual ways and means,
- any residual solvent, non microencapsulated active ingredient and/or surfactant are removed as necessary.

2. Process according to Claim 1,
**characterised in that**
acetone, ethanol, C₁-C₄-alkylacetates, triacetin, triethylcitrate, C₁-C₄-alkylformiates and C₁-C₄-alkyllactates or mixtures thereof are used as halogen-free solvents.

3. Process according to either of Claims 1 or 2,
**characterised in that**
methylacetate, ethylacetate, isopropylacetate and propylformiate are used as halogen-free solvents.

4. Process according to any of Claims 1 to 3,
**characterised in that**
a phosphate buffer solution, a citrate buffer solution or a tris(hydroxymethyl)aminomethane buffer solution is used as the buffer solution.

5. Process according to any of Claims 1 to 4,
**characterised in that**
the buffered solution of the active ingredient has a pH between 6.0 and 8.0.

6. Process according to any of Claims 1 to 4,
**characterised in that**
Resomer ®, and preferably Resomer ® RG-756 or Resomer® RG-858, is used as the polymer.

7. Process according to any of Claims 1 to 6,
**characterised in that**
human serum albumin, peptides, proteins, interferones, Betaseron ®, insulin, LHRH-antagonists, or their analogues are used as the active ingredient.

8. Process according to any of Claims 1 to 7,
**characterised in that**
a) DesA(2) Nal-beta Ala-DCpa-DPal-Ser-Tyr-DCit-Leu-Arg-Pro-DAla-NH₂
b) DesA(2) Nal-Gly-DCpa-DPal-Ser-Tyr-DCit-Leu-Arg-Pro-DAla-NH₂
or
c) Ac-DNal-DCpa-DPal-Ser-Tyr-DLys(Mor)-Leu-Lys(Mor) Pro-DAla-NH₂
are used as the active ingredient.

9. Process according to any of Claims 1 to 8,
**characterised in that**
the solvent or solvent mixture and any active ingredient and/or surfactant that may have adhered to the particle surface are removed by "cross-flow" filtration.

10. Morphologically uniform microcapsules with a loading level between 3 and 30 weight-% and a diameter of 200 nm to 500 µm, prepared by the process according to Claims 1 to 9.

11. Microcapsules according to Claim 10, with a diameter between 0.2 and 8 µm.

## Revendications

1. Procédé de production de microcapsules morphologiquement unitaires en polymères ou copolymères biodégradables, qui contiennent un peptide, une protéine ou d'autres substances solubles dans l'eau biologiquement actives, selon la méthode de "transition de phase induite", caractérisé en ce que :
◆ on dissout des polyesters d'acides hydroxycarboniques ou des copolymères bloc de polyesters d'acide hydroxycarbonique et de polyéthylène glycol ou un mélange de solvants, dans un solvant exempt d'halogènes non miscible ou partiellement miscible à l'eau,
◆ on disperse dans cette solution la solution tamponnée d'ingrédient actif, qui présente un pH compris entre 6,0 et 8,0,
◆ on ajoute à cette émulsion E/H une solution aqueuse contenant une substance tensioactive ou un mélange de substances tensioactives,
◆ ensuite on élimine le solvant ou le mélange de solvants selon l'art et la manière habituels et
◆ au choix on élimine le solvant résiduel, l'ingrédient actif non encapsulé et/ou la substance tensioactive.

2. Procédé selon la revendication 1, caractérisé en ce que, on utilise comme solvant exempt d'halogènes l'acétone, l'éthanol, des acétates d'alkyle C₁-C₄, la triacétine, le citrate de triéthyle, des formiates d'alkyle C₁-C₄, des lactates d'alkyle C₁-C₄, ou des mélanges de ceux-ci.

3. Procédé selon l'une des revendications 1 ou 2, caractérisé en ce que, on utilise comme solvant exempt d'halogènes de l'acétate de méthyle, de l'acétate d'éthyle, de l'acétate d'isopropyle et du formiate de propyle.

4. Procédé selon l'une des revendications 1 à 3, caractérisé en ce que, on utilise comme solution tampon une solution de tampon phosphate, une solution de tampon citrate ou une solution de tampon Tris (hydroxyméthyl) aminométhane.

5. Procédé selon l'une des revendications 1 à 4, caractérisé en ce que, la solution tamponnée d'ingrédient actif a un pH compris entre 6,0 et 8,0.

6. Procédé selon l'une des revendications 1 à 4, caractérisé en ce que, on utilise comme polymère le Resomer®, de préférence le Resomer® RG-756 ou le Resomer® RG-858.

7. Procédé selon l'une des revendications 1 à 6, caractérisé en ce que, on utilise comme ingrédient actif la sérumalbumine humaine, des peptides, des protéines, des interférons, le Betaseron®, l'insuline, des antagonistes de la LHRH ou des analogues de ceux-ci.

8. Procédé selon l'une des revendications 1 à 7, caractérisé en ce que, on utilise comme ingrédients actifs
a) le DesA (2)Nal-beta Ala-DCpa-DPal-Ser-Tyr-DCit-Leu-Arg-Pro-DAla-NH₂
b) le DesA (2) Nal-Gly-DCpa-DPal-Ser-Tyr-DCit-Leu-Arg-Pro-DAla-NH₂ ou
c) le Ac-DNal-DCpa-DPal-Ser-Tyr-DLys(Mor)-Leu-Lys (Mor) Pro-DAla-NH₂

9. Procédé selon l'une des revendications 1 à 8, caractérisé en ce que l'élimination du solvant ou du mélange de solvants, ainsi que le cas échéant les ingrédients actifs et/ou les tensioactifs liés à la surface des particules est effectuée par une filtration "cross-flow".

10. Microcapsules morphologiquement unitaires avec un taux de charge compris entre 3 et 30 % en poids et un diamètre compris entre 200 nm et 500 *µ*m produites par le procédé selon les revendications 1 à 9.

11. Microcapsules selon la revendication 10, d'un diamètre compris entre 0,2 et 8 *µ*m.
